# FASCICULE DE BREVET EUROPEEN

(11) **EP 3 600 370 B1**
(45) Date de publication et mention de la délivrance du brevet: **03.04.2024**
(21) Numéro de dépôt: 18711130.7
(22) Date de dépôt: 20.03.2018
(51) Int. Cl.: A61K 36/48, A61P 13/08

(54) **UTILISATION DE L'OLEORESINE DE COPAIFERA DANS LES PATHOLOGIES DE LA PROSTATE**
VERWENDUNG VON COPAIFERA-OLEOHARZ BEI PATHOLOGIEN DER PROSTATA
USE OF COPAIFERA OLEORESIN IN PATHOLOGIES OF THE PROSTATE

(30) Priorité: 20.03.2017 FR 1752284
(43) Date de publication de la demande: 05.02.2020
(73) Titulaire: PIERRE FABRE MEDICAMENT, 81500 Lavaur (FR)
(72) Inventeur: FIORINI-PUYBARET, Christel, 31500 Toulouse (FR)
(74) Mandataire: Regimbeau
(86) Numéro de dépôt international: PCT/EP2018/057076
(87) Numéro de publication internationale: WO 2018/172380

(56) Documents cités:
- RATI LAMA ET AL: "Bioassay guided identification of small chaperone proteins [alpha]-crystallin and Hsp27 inhibitors from Copaiba oil", PHYTOCHEMISTRY LETTERS, vol. 10, 1 décembre 2014 (2014-12-01), pages 65-75, XP055422813, AMSTERDAM, NL ISSN: 1874-3900, DOI: 10.1016/j.phytol.2014.08.006
- LIDIAM MAIA LEANDRO ET AL: "Chemistry and Biological Activities of Terpenoids from Copaiba (Copaifera spp.) Oleoresins", MOLECULES ONLINE, vol. 17, no. 12, 30 décembre 2012 (2012-12-30), pages 3866-3889, XP055313075, DE ISSN: 1433-1373, DOI: 10.3390/molecules17043866
- DE MATOS GOMES N ET AL: "Antineoplasic activity of Copaifera multijuga oil and fractions against ascitic and solid Ehrlich tumor", MEDICINAL & AROMATIC PLANTS ABSTRACTS, SCIENTIFIC PUBLISHERS, SCIENTIFIC PUBLISHERS, NEW DELHI - INDIA, vol. 31, no. 4, 1 août 2009 (2009-08-01), XP018025493, ISSN: 0250-4367
- VEIGA ET AL: "Chemical composition and anti-inflammatory activity of copaiba oils from Copaifera cearensis Huber ex Ducke, Copaifera reticulata Ducke and Copaifera multijuga Hayne-A comparative study", JOURNAL OF ETHNOPHARMACOLOGY, ELSEVIER IRELAND LTD, IE, vol. 112, no. 2, 19 mai 2007 (2007-05-19), pages 248-254, XP022085297, ISSN: 0378-8741, DOI: 10.1016/J.JEP.2007.03.005

## Description

La présente invention se réfère à une utilisation de l'oléorésine de Copaifera pour prévenir et/ou traiter l'hyperplasie bénigne de la prostate.

Le genre *Copaifera* compte 35 espèces, toutes des arbres d'Amérique tropicale, c'est-à-dire au Mexique, le nord de l'Argentine et principalement au Brésil. Sur le territoire on dénombre plus de vingt espèces, les plus abondantes étant *C*. *officinalis, C. reticulata, C. multijuga. Copaifeira officinalis* est un arbre que l'on retrouve essentiellement au Brésil, en Colombie et au Venezuela. C'est un arbre d'une hauteur jusqu'à 25 m, à bois brun-rougeâtre. Les feuilles sont composées, paripennées avec 2 à 10 folioles, alternes ou sub-opposées, apiculées et inégalement arrondies à la base. Elles sont longues de 3 à 8 cm et larges de 2 à 4 cm. Les fleurs blanches, généralement sessiles sont groupées en inflorescences de 7 à 14 cm. Les fruits sont des petites gousses renflées à maturité de diamètre 20-25 mm glabre, apiculées renfermant une graine ovoïde.

L'oléorésine est une substance obtenue à l'aide d'incisions de l'écorce de plusieurs espèces de *Copaifera.* Localisée dans des canaux sécréteurs anastomosés du bois secondaire et de la moelle, son extraction nécessite donc des incisions très profondes du tronc ce qui lui permet de s'écouler naturellement de l'arbre.

Après distillation à la vapeur, ou hydro distillation l'oléorésine permet d'obtenir l'huile essentielle de Copaïba réputée dans la parfumerie.

L'oléorésine de Copaïba a été utilisée en médecine à partir du XVI^{ème} siècle par les indigènes du Brésil. Elle a une longue histoire d'utilisation dans la médecine traditionnelle brésilienne, pour soigner les blessures et faire disparaitre les cicatrices, fébrifuge, antiseptique des voies urinaires, contre la leucorrhée et la blennorragie. Considéré comme un tonique général, ses indications étaient les suivantes : maladie vénérienne, maladie respiratoire, asthme, rhumatisme, lésions dermiques secondaires, ulcères. A dose faible, c'est un stimulant d'action directe sur l'estomac. L'oléorésine de Copaïba diminue la sécrétion excessive de mucus provoquée par des inflammations. Aujourd'hui, l'oléorésine de Copaïba est vendue en gélules dans les pharmacies du Brésil où elle est indiquée pour tous les types d'inflammations internes et les ulcères de l'estomac. En application locale, c'est un cicatrisant puissant à la fois antiseptique et anti-inflammatoire, il aide à la cicatrisation des plaies les plus difficiles. L'oléorésine de Copaïba serait très efficace sur les douleurs articulaires, les entorses bénignes, les hématomes, les tendinites. L'oléorésine est appliquée directement sur la peau. L'oléorésine est également utilisée sous forme d'huile de massage pour les muscles et les articulations douloureuses ou enflammées.

L'art antérieur divulgue que des protéines chaperonnes (telle qu'HSP27) contribuent à une résistance aux chimiothérapies par agents chimiques au sein des tissus cancéreux et il est ainsi décrit que l'acide hardwikiique d'huile de copaifera inhibe la protéine HSP27. Les recherches sur ce sujet visent à identifier d'autres inhibiteurs de chaperonnes dans l'huile de copaifera (Phytochemistry Letters, 10 (2014) ; 65-75). Un tel document indique que la fraction non acide et une seule sous-fraction acide (sans acides diterpéniques ou esters d'acides diterpéniques) de l'huile ont une action anti-proliférative. Les autres sous fractions (ie acides) démontrent une action anti-chaperonne. Ce document vise ainsi un cas de figure bien particulier, à savoir une combinaison d'huile de copaifera avec un traitement chimio thérapeutique par agent chimique et ce afin de lutter contre la résistance des cellules cancéreuses audit traitement par l'agent chimique. Ce document conclut seulement à une activité de l'acide hardwikiique d'huile de copaifera en combinaison avec un agent antinéoplasique, qui est lui l'agent anti-cancéreux, afin d'inhiber les protéines chaperonnes qui représentent des agents contribuant à une résistance audit agent anti-cancéreux.

L'oléorésine de Copaïba, distillée ou non, est également utilisée en cosmétique, dans la fabrication de savons, de bains moussants, détergents et crèmes, et comme fixateur en parfumerie. L'oléorésine est parfois utilisée comme aromatisant dans le domaine alimentaire. L'oléorésine de Copaïba est également utilisée comme matériau d'artiste, en particulier dans les recettes de peinture à l'huile et dans la céramique de décoration.

L'oléorésine est un liquide incolore et peu consistant, il acquiert une consistance oléagineuse et une couleur jaune verdâtre avec le temps. Sa consistance et sa couleur varient un peu, suivant l'arbre dont il provient et suivant l'huile essentielle qui s'y trouve. Son odeur est forte et désagréable, sa saveur est amère et âcre. L'oléorésine est insoluble dans l'eau, soluble en totalité dans l'alcool et dans l'éther

L'oléorésine de *C*. *officinalis* est constituée de 2 fractions qui se distinguent par leur volatilité ; chacune des fractions étant caractérisée par des composés chimiques distincts :
- Une huile essentielle « volatile » représentant 50-90% de l'oléorésine, qui est majoritairement composée de sesquiterpènes. Parmi ces sesquiterpènes, on distingue majoritairement du germacrène D, (E)-β-caryophyllène, β-et δ-élémène, α-ylangenea, α-gurjunène, α-humulène. Les sesquiterpènes minoritaires sont les suivants α-cubébène, α-copaène, 7-epi-sesquithujène, cis- et trans-α-bergamotène, sesquisabinène-A et B, 4αH,10αH-guaia-1(5),6-diène, allo-aromadendrène, γ-uurolène, α-amorphène, β-selinène, bicycliosesquiphyllandrène, α-muurolène, β-bisabolène, γ-cadinène, δ-cadinène, cis-calamène, zonarène, cakina-1'4-diène, α-cadinène, α-calacorène, selina-3,7(11)-diène, germacrène B. Cette huile volatile est très limpide, incolore, d'une odeur et d'une saveur très prononcées.
- Une fraction « non volatile » représentant 10-50% de l'oléorésine, qui est composée majoritairement des acides diterpéniques et/ou esters d'acides diterpéniques suivants : l'acide copalique, l'acide copaiférolique, l'ester diméthylique de l'acide agathendioique, l'acide agathique, l'ester méthylique de l'acide 3beta-hydroxyanticopalique, l'acide hardwickiique, l'acide 7 alpha acetoxyhardwickiique. Ce résidu de distillation est un liquide visqueux, d'odeur aromatique, de couleur marron foncé.

Les inventeurs de façon toute à fait surprenante ont mis en évidence que l'oléorésine de Copaifera présente une activité anti-5α-réductase particulièrement intéressante.

Le rôle des inhibiteurs de la 5α-réductase est établi dans la physiopathologie de la prostate (Rittmaster, Journal of Andrology, vol 18, n°6, 1997), notamment dans l'hyperplasie bénigne de la prostate et le cancer de la prostate.

La prostate est la glande exocrine la plus volumineuse de l'appareil urogénital masculin. Elle est située au croisement des voies génitales et urinaires. Avec les vésicules séminales, la prostate joue un rôle essentiel dans la synthèse et l'émission du liquide spermatique. Elle contribue plus indirectement au cycle miction-continence par sa composante musculaire lisse. Enfin, la prostate est entourée par des pédicules vasculo-nerveux participant à la réponse sexuelle masculine. Avec le vieillissement, les modifications anatomiques de la glande prostatique sont parfois à l'origine de troubles urinaires et de dysfonctions sexuelles responsables d'une diminution de la qualité de vie.

La prostate peut être le siège de trois principales maladies, la prostatite, l'adénome de la prostate ou l'hypertrophie bénigne de la prostate et le cancer de la prostate. Cependant l'enzyme 5α-réductase n'est impliquée à ce jour que dans l'hypertrophie bénigne de la prostate et le cancer de la prostate.

L'hypertrophie bénigne de la prostate est une pathologie fréquente favorisée par le vieillissement et liée au développement d'un adénome prostatique responsable d'un obstacle chronique à la vidange vésicale. L'hypertrophie bénigne de la prostate se caractérise par une augmentation de la taille de la prostate. Une prostate volumineuse comprime l'urètre tout en faisant pression sur la vessie, ce qui engendre un besoin fréquent d'uriner et divers problèmes de miction, débit plus faible et intermittent, douleur, etc. Presque tous les hommes sont sujets à l'hypertrophie bénigne de la prostate, en vieillissant. En effet, plus de 50% des hommes âgés de 60 ans en sont atteints, et 90% de ceux de plus de 80 ans. Cependant, tous n'en souffrent pas, en effet un homme sur deux est incommodé par des symptômes urinaires. Les causes de cette pathologie ne sont pas clairement établies, il existe probablement une prédisposition héréditaire, mais d'autres facteurs entrent en jeu. Cette pathologie n'est cependant pas à prendre à la légère, car il y a plusieurs complications possibles comme des infections urinaires, une rétention aiguë d'urine, des calculs dans la vessie et des dommages aux reins.

Le cancer de la prostate est une pathologie fréquente qui représente le deuxième cancer chez l'homme dans les pays industrialisés. Il existerait une prédisposition génétique. La plupart des cancers de la prostate évoluent très lentement. Souvent la tumeur demeure localisée dans la prostate et a des effets limités sur la santé, provoquant parfois des troubles urinaires ou érectiles. Il peut arriver cependant que certains cancers de la prostate évoluent et s'étendent plus rapidement. L'adénocarcinome est la forme la plus courante de cancer de la prostate. Il représente environ 95% des cas. La gravité du cancer dépend de l'étendue de la tumeur et du type de cellules cancéreuses.

La testostérone favorise la croissance prostatique grâce à l'un de ses métabolites, la dihydrotestostérone. La testostérone est transformée en dihydrotestostérone sous l'action de la 5α-réductase. La dihydrotestostérone a une affinité de liaison élevée pour le récepteur des androgènes. En comparaison avec la testostérone, la dihydrotestostérone stimule de 2 à 10 fois l'activité transcriptionnelle des cellules prostatiques. *Ex vivo* la dihydrotestostérone a un effet stimulant beaucoup plus puissant sur la croissance tumorale de la prostate que la testostérone. Chez les hommes ayant une hyperplasie bénigne de la prostate symptomatique, l'inhibition de de la 5α-réductase diminue le volume de la prostate, améliore les symptômes et le débit urinaire et réduit le risque de rétention urinaire aiguë.

Il existe 2 isoformes de la 5α-réductase, la 5αR1 et la 5αR2, qui sont codées respectivement par 2 gènes distincts, SRD5A1 et SRD5A2, situés sur des chromosomes séparés. L'isoenzyme 5αR2 se produit principalement dans les tissus reproducteurs masculins, les vésicules séminales, l'épididyme et la prostate. Alors que l'isoenzyme 5αR1 est détectée principalement dans le foie et la peau. La 5αR2 est présente en concentrations élevées dans le tissu prostatique et a une affinité élevée pour la testostérone. Le finastéride est un puissant inhibiteur de la 5αR2, avec une concentration moyenne inhibitrice IC₅₀ de 69nM mais qui est beaucoup moins efficace pour inhiber la 5αR1, IC₅₀ de 360nM. En revanche, le dutastéride inhibe à la fois la 5αR1 et la 5αR2 de façon similaire, IC₅₀ de 6 et 7nM, respectivement. Le dutastéride semble plus efficace que le finastéride, respectivement 94,7 vs 70,8% pour réduire les niveaux moyens de dihydrotestostérone sérique. Au niveau intraprostatique, une réduction plus importante de dihydrotestostérone a été observée avec le dutastéride, suggérant que 5αR1 semble contribuer à la synthèse de dihydrotestostérone intrapostatique. Le rôle de la 5α-réductase dans l'inhibition du cancer de la prostate a été rapporté dans des modèles animaux.

Selon un premier mode de réalisation, l'invention concerne une oléorésine de Copaifera pour son utilisation en tant que médicament destiné au traitement et/ou à la prévention de l'hyperplasie bénigne de la prostate.

Dans un second mode de réalisation, l'invention vise une oléorésine de Copaifera pour son utilisation selon le premier mode de réalisation, caractérisée en ce que l'oléorésine provient de l'espèce de Copaifera choisie parmi le groupe constitué de C. officinalis, C. multijuga, C. reticulata.

Dans un troisième mode de réalisation, l'invention vise la fraction d'acides diterpéniques et/ou esters d'acides diterpéniques de l'oléorésine pour son utilisation selon l'un des modes de réalisations 1 ou 2, caractérisée en ce qu'elle contient au moins 90% d'acides diterpéniques et/ou esters d'acides diterpéniques en poids.

Un quatrième mode de réalisation de l'invention vise une composition pharmaceutique caractérisée en ce qu'elle contient comme agent actif, une oléorésine de Copaifera selon l'un des modes de réalisation 1 ou 2, ou une fraction selon le troisième mode de réalisation, et au moins un excipient pharmaceutiquement acceptable, pour son utilisation comme médicament destiné au traitement et/ou la prévention de l'hyperplasie bénigne de la prostate. De manière avantageuse, l'oléorésine ou la fraction est le seul agent actif de ladite composition.

Un cinquième mode de réalisation de l'invention concerne une composition pharmaceutique pour son utilisation selon le quatrième mode de réalisation, caractérisée en ce que l'oléorésine provient de l'espèce de Copaifera choisie parmi le groupe constitué de C. officinalis, C. multijuga, C. reticulata. Un sixième mode de réalisation de l'invention concerne une composition pharmaceutique pour son utilisation selon l'un des modes de réalisation 4 ou 5, caractérisée en ce qu'elle contient comme agent actif une fraction selon le troisième mode de réalisation.

Un septième mode de réalisation de l'invention concerne encore une composition pharmaceutique pour son utilisation selon l'un des modes de réalisation 4 à 6, caractérisée en ce qu'elle se présente sous une forme adaptée à une administration par voie orale ou intraveineuse.

De manière avantageuse, l'oléorésine ou la fraction selon la présente invention représente le seul principe actif destiné au traitement et/ou la prévention de l'hyperplasie bénigne de la prostate dans les compositions pharmaceutiques pour leur utilisation selon l'invention.

L'utilisation d'une oléorésine de Copaifera ou d'une fraction telle que définie selon l'un des modes de réalisation précédents pour la fabrication d'un médicament destiné au traitement et/ou la prévention de l'hyperplasie bénigne de la prostate est décrite.

Une méthode de traitement et/ou de prévention de l'hyperplasie bénigne de la prostate comprenant l'administration, à un sujet qui le nécessite, d'une composition pharmaceutique comprenant, ou consistant en, une oléorésine de Copaifera ou une fraction selon l'un des modes de réalisation précédents, comme agent actif, et au moins un excipient pharmaceutiquement acceptable est décrite.

De manière avantageuse, l'oléorésine ou la fraction est le seul principe actif anticancéreux de la composition.

Par « oléorésine de Copaifera » on entend au sens de la présente invention un exsudat d'arbre(s) de l'espèce Copaifera comprenant une fraction volatile principalement constituée de composés sesquiterpéniques et une fraction non volatile principalement constituée d'acides diterpéniques et/ou esters d'acides diterpéniques.

Par « fraction d'acides diterpéniques et/ou esters d'acides diterpéniques de l'oléorésine de Copaifera » on entend au sens de la présente invention la fraction « non volatile » de l'oléorésine obtenue après élimination totale ou partielle, de préférence élimination totale, de l'huile essentielle, notamment par hydrodistillation. Cette fraction non volatile comprend au moins 80% et de préférence entre 80-90% en poids d'acides diterpéniques et/ou esters d'acides diterpéniques.

Le mélange d'acides diterpéniques et/ou esters d'acides diterpéniques peut aussi être obtenu à partir d'une oléorésine de Copaifera, ou de la fraction « non volatile » de l'oéorésine de Copaifera, notamment par extraction liquide-liquide jusqu'à obtenir un mélange présentant une concentration en acides diterpéniques et/ou esters d'acides diterpéniques comprise entre 50 et 100%, particulièrement entre 60 et 100%, plus particulièrement encore entre 80 et 100% en poids d'acides diterpéniques et/ou esters d'acides diterpéniques par rapport au poids total de la fraction liquide obtenue après extraction et élimination du solvant d'extraction. Cette fraction liquide obtenue après extraction et élimination du solvant représente ledit mélange.

Le mélange d'acides diterpéniques et/ou esters d'acides diterpéniques comprend au moins 2, voire au moins 3, voire au moins 4, voire au moins 5, voire au moins 6, voire au moins 7 acides diterpéniques et/ou esters d'acides diterpéniques choisis dans le groupe constitué par : l'acide copalique, l'acide copaiférolique, l'ester diméthilique de l'acide agathendioique, l'acide agathique, l'ester méthylique de l'acide 3beta-hydroxyanticopalique, l'acide hardwickiique, l'acide 7 alpha acetoxyhardwickiique.

**Tableau 1 : formules brutes, développées et numéro CAS des différents acides diterpéniques et/ou esters d'acides diterpénqiues**

| Nom | Formule brute | m/z | Formule développée |
|---|---|---|---|
| Acide copalique | C₂₀H₃₂O₂ | 304 | 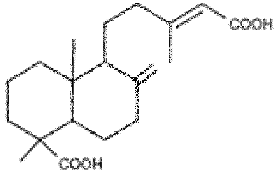 |
| Acide copaiférolique | C₂₀H₃₂O₃ | 320 | 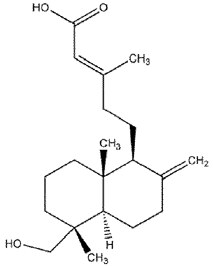 |
| Ester diméthylique de l'acide agathendioique | C₂₂H₃₄O₄ | 362 | 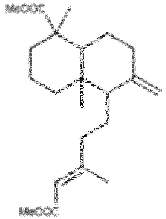 |
| Acide agathique | C₂₀H₃₀O₄ | 334 | 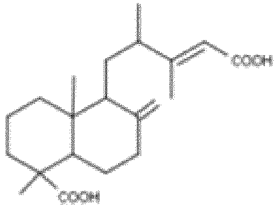 |
| Ester méthylique de l'acide 3beta-hydroxyanticopa lique | C₂₀H₃₀O₄ | 334 | 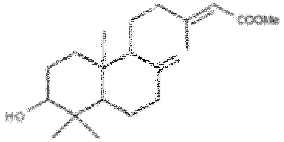 |
| Acide hardwickiique | C₂₀H₂₈O₃ | 316 | 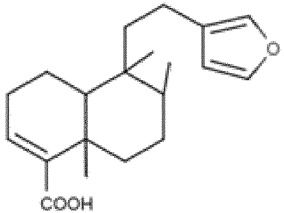 |
| Acide 7 alpha acetoxyhardwick iique | C₂₂H₃₀O₅ | 374 | 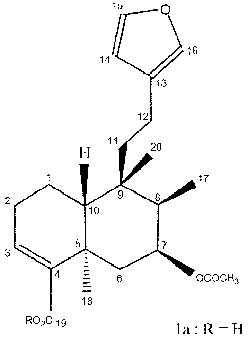 |

De préférence, le mélange d'acides diterpéniques et/ou esters d'acides diterpéniques comprend tous les acides diterpéniques et/ou esters d'acides diterpéniques suivants : l'acide copalique, l'acide copaiférolique, l'ester diméthilique de l'acide agathendioique, l'acide agathique, l'ester méthylique de l'acide 3beta-hydroxyanticopalique, l'acide hardwickiique, l'acide 7 alpha acetoxyhardwickiique.

Dans un mode de réalisation de l'invention, l'oléorésine comprend, en pourcentage en poids, entre 7,5 et 40% des 7 acides diterpéniques et/ou esters d'acides diterpéniques cités ci-dessus, voire de préférence 10 à 30%.

Dans un mode de réalisation de la présente invention, l'oléorésine peut être une oléorésine enrichie en les acides diterpéniques et/ou esters d'acides diterpéniques et la teneurs en les 7 acides diterpéniques et/ou esters d'acides diterpéniques d'une telle oléorésine enrichie sera comprise entre 48 et 90%, de préférence entre 60 et 90%, de préférence encore entre 60 et 85%.

Par l'expression « résine enrichie », on entend au sens de la présente invention que l'oléorésine a fait l'objet d'un traitement par un procédé visant à concentrer la teneur en ces 7 acides diterpéniques et/ou esters d'acides diterpéniques par rapport aux autres composés et molécules de cette oléorésine. Ainsi les ratios entre acides diterpéniques et/ou esters d'acides
diterpéniques et autres molécules sont modifiés dans le sens d'une augmentation et représente des ratios différents des produits naturels ou des produits extraits de l'arbre.

**Tableau 2 : teneur massique des principaux acides diterpéniques et/ou esters d'acides diterpéniques**

| Dénomination | Teneur massique dans l'oléorésine (%) | Teneur massique en fraction diterpénique (%) |
|---|---|---|
| Acide copalique | 3 à 13 | 15 à 50 |
| Acide hardwickiique | 0,5 à 1,5 | 2 à 15 |
| Acide copaiférolique | 1 à 6 | 10 à 30 |
| Acide agathique et ester méthylique de l'acide 3beta-hydroxyanticopalique | 1 à 6 | 8 à 30 |
| Ester diméthylique de l'acide agathendioique | 1 à 6 | 10 à 30 |
| Acide 7-alpha acetoxyhardwickiique | 1 à 8 | 3 à 15 |

La présente invention concerne l'oléorésine de Copaifera pour son utilisation en tant que médicament destiné au traitement et/ou à la prévention de l'hyperplasie bénigne de la prostate.

Selon un mode particulier, l'oléorésine provient de l'espèce de Copaifera choisie parmi le groupe constitué par Copaifera officinalis, Copaifera multijuga ou Copaifera reticulata, pour son utilisation en tant que médicament destiné au traitement et/ou à la prévention de l'hyperplasie bénigne de la prostate. D'une façon préférée l'oléorésine provient de l'espèce Copaifera officinalis.

Selon un mode de réalisation de l'invention tout aussi avantageux, seule une fraction d'acides diterpéniques et/ou esters d'acides diterpéniques de l'oléorésine est utilisée en tant que médicament destiné au traitement et/ou à la prévention de l'hyperplasie bénigne de la prostate. La fraction d'oléorésine utilisée est constituée d'au moins 90% d'acides diterpéniques et/ou esters d'acides diterpéniques, de façon avantageuse d'au moins 92% d'acides diterpéniques et/ou esters d'acides diterpéniques et de façon encore plus avantageuse d'au moins 95% d'acides diterpéniques et/ou esters d'acides diterpéniques en poids.

L'oléorésine de Copaifera est utile pour la fabrication d'un médicament destiné à prévenir et/ou traiter l'hyperplasie bénigne de la prostate.

L'oléorésine de Copaifera officinalis, C. multijuga ou bien C. reticulata est utile pour la fabrication d'un médicament destiné à prévenir et/ou traiter l'hyperplasie bénigne de la prostate.

L'utilisation de l'oléorésine de Copaifera officinalis, est utile pour la fabrication d'un médicament destiné à prévenir et/ou traiter l'hyperplasie bénigne de la prostate.

Une fraction d'acides diterpéniques et/ou esters d'acides diterpéniques de l'oléorésine de Copaifera, constituée d'au moins 90% d'acides diterpéniques et/ou esters d'acides diterpéniques, de préférence d'au moins 92% d'acides diterpéniques et/ou esters d'acides diterpéniques, et d'une façon encore préférée d'au moins 95% d'acides diterpéniques et/ou esters d'acides diterpéniques en poids, est utile pour la fabrication d'un médicament destiné à prévenir et/ou traiter l'hyperplasie bénigne de la prostate.

Par « traitement » selon la présente invention, on entend l'inhibition de l'évolution, plus particulièrement la régression, préférentiellement la disparition de l'hyperplasie ou tumeur de la prostate.

Par « prévention » selon la présente invention, on entend empêcher ou retarder l'apparition de l'hyperplasie de la prostate.

Le traitement ou la prévention selon l'invention s'entend chez l'homme ou l'animal.

La présente invention concerne en outre une composition pharmaceutique comprenant de l'oléorésine de Copaifera et au moins un excipient pharmaceutiquement acceptable.

Dans la présente invention, on entend désigner par « pharmaceutiquement acceptable » ce qui est utile dans la préparation d'une composition pharmaceutique, qui est généralement sûr, non toxique et ni biologiquement ni autrement non souhaitable et qui est acceptable pour une utilisation pharmaceutique chez l'homme.

Quand utilisé ici, le terme « excipient pharmaceutiquement acceptable » inclut tout adjuvant ou excipient, tels que des solvants, des solubilisants, des conservateurs, des émulsionnants, des agents de consistance, des agents d'étalement, des agents fixateurs d'eau, des colorants, des arômes, des édulcorants, l'utilisation de ces excipients est bien connue de l'homme du métier.

La présente invention concerne en outre une composition pharmaceutique pour son utilisation en tant que médicament.

La présente invention concerne également une composition pharmaceutique caractérisée en ce qu'elle contient comme agent actif une oléorésine de Copaifera selon l'invention ou une fraction d'acides diterpéniques et/ou esters d'acides diterpéniques d'oléorésine de Copaifera selon l'invention, et au moins un excipient pharmaceutiquement acceptable, pour son utilisation comme médicament destiné au traitement et/ou la prévention de l'hyperplasie bénigne de la prostate.

Selon un mode particulier de l'invention, la composition contient comme agent actif une oléorésine provenant de l'espèce de Copaifera choisie parmi le groupe constitué de C. officinalis, C. multijuga, C. reticulata et au moins un excipient pharmaceutiquement acceptable, pour son utilisation comme médicament destiné au traitement et/ou la prévention de l'hyperplasie bénigne de la prostate.

Selon un autre mode de réalisation de l'invention, la composition contient comme agent actif une fraction d'acides diterpéniques et/ou esters d'acides diterpéniquesde l'oléorésine qui contient au moins 90% d'acides diterpéniques et/ou esters d'acides diterpéniques en poids, préférentiellement au moins 92% d'acides diterpéniques et/ou esters d'acides diterpéniques et de façon encore préférée au moins 95% d'acides diterpéniques et/ou esters d'acides diterpéniques en poids et au moins un excipient pharmaceutiquement acceptable, pour son utilisation comme médicament destiné au traitement et/ou la prévention de l'hyperplasie bénigne de la prostate.

Les compositions pharmaceutiques selon la présente invention peuvent être formulées sous une forme adaptée pour l'administration aux mammifères, y compris l'homme. La posologie varie selon le traitement et selon l'affection en cause. Ces compositions sont réalisées de façon à pouvoir être administrées par voie orale, sublinguale, sous-cutanée, intramusculaire, intraveineuse, transdermique, locale ou rectale. Dans ce cas, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, topique, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique, la silice ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylènes glycols.

Pour une administration parentérale (intraveineuse, intramusculaire etc.), intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

Avantageusement, la composition pharmaceutique selon la présente invention se présente sous une forme adaptée à une administration par voie orale ou intraveineuse.

La composition selon la présente invention peut être administrée en association, de manière simultanée, séparée ou étalée dans le temps, avec une prostatectomie, une radiothérapie, et/ou une hormonothérapie, en vue du traitement ou de la prévention du cancer de la prostate.

Les exemples suivants illustrent l'invention sans en limiter la portée.

### Exemple 1 : Effets de différents composés sur l'activité de 5α-réductase de fibroblastes issus de follicules humains de papille dermique.

L'objectif de cette étude était d'évaluer une activité inhibitrice éventuelle de différents composés sur la 5α-réductase.

### Matériel et méthodes

L'étude est réalisée sur des cellules humaines issues de follicule de papilles dermiques de donneurs. Ce modèle est intéressant, en effet les papilles dermiques présentent l'isoforme 5αR2 comme dans le tissu prostatique. Les cellules sont ensemencées dans des plaques de 24 puits et mises en culture pendant 24h dans un milieu de culture DMEM supplémenté en L-glutamine (2mM), de pénicilline (50U/ml), de streptomycine (50pg/ml) et de Fetal Calf Serum (10%) dans des conditions de cultures standards (37°C et 5% CO₂). Le milieu de culture est ensuite remplacé par un milieu d'analyse DMEM supplémenté en L-glutamine (2mM), de pénicilline (50U/ml), de streptomycine (50pg/ml) et de Fetal Calf Serum (1%). Ce milieu d'analyse peut contenir ou pas (conditions contrôles) les produits à tester et un composé servant de référence le finastéride (10µM) pendant 24h de pré-incubation. Les cellules étaient ensuite traitées avec un milieu d'analyse contenant de la testostérone [C¹⁴] et contenant ou pas (conditions contrôles) les produits à tester ou de référence, et les cellules étaient incubées pendant 24h dans ces conditions. Après incubation, les surnageants étaient collectés pour l'analyse du métabolisme de la testostérone. Toutes les expériences ont été réalisées 3 fois. Les molécules stéroïdes étaient extraites des surnageants avec un mélange chloroforme/méthanol. La phase organique était collectée et les différentes espèces moléculaires (métabolites de la testostérone) étaient séparées par chromatographie sur couche mince en utilisant un système de solvants contenant du dichlorométhane, de l'éthylacétate et du méthanol. Une autoradiographie était réalisée sur la chromatographie et la testostérone transformée était estimée par analyse densitométrique.

Ainsi la métabolisation de la testostérone en dihydrotestostérone rend compte de l'activité 5α-réductase, elle est évaluée par le ratio dihydrotestostérone/testostérone.

### Résultats

Un premier groupe d'expériences permet de mettre en évidence les effets de l'oléorésine de Copaifera officinalis (tableau 3 ci-dessous). De façon surprenante, les inventeurs ont mis en évidence une activité significative et reproductible de l'inhibition de la 5α-réductase par l'oéorésine de Copaifera officinalis, cette inhibition apparaît même concentration-dépendante. L'inhibition importante de cette enzyme par le finastéride permet de bien valider l'ensemble de ces expériences.

**Tableau 3 : Effets de l'oléorésine de Copaifera officinalis et du finastéride sur la métabolisation de la testostérone / la production de dihydrotestostérone (activité 5α-réductase ; n=3)**

| Contrôle | Finastéride | Oléorésine (C. officinalis) | |
|---|---|---|---|
| | 10 µM | 10 µg/ml | 30 µg/ml |
| 100 | -77% | -15% | -27% |
| | ** P<0,01 | * P<0,05 | ** P<0,01 |

| | | | |
|---|---|---|---|
| L'étude statistique est réalisée versus le groupe contrôle (test de Dunnett). L'oléorésine testée a été préparée selon la méthode décrite dans l'exemple 2. | | | |

A titre de comparaison, un extrait de Serenoa repens a également été testé dans une de ces expériences. L'extrait de Serenoa repens, issu du fruit du palmier nain est le phytonutriment le plus étudié et le plus souvent prescrit seul ou en association, particulièrement dans le traitement de l'hyperplasie bénigne de la prostate (Gordon AE, Am. Fam. Physician, 67(06), 1281-1283, 2003). A 10 pg/ml l'extrait de serenoa repens n'induit pas d'inhibition significative de la 5α-réducatse en revanche à 20 µg/ml cet extrait induit une inhibition de 23% atteignant la significativité statistique (p<0,05 versus contrôle).

Une seconde série d'expériences a été menée pour évaluer si l'activité inhibitrice sur 5α-réductase était portée par la fraction non volatile ou plutôt par la fraction volatile correspondant à l'huile essentielle. Les résultats sont résumés dans le tableau 4 ci-dessous. La préparation des fractions volatile et non volatile est réalisée selon la méthode décrite dans l'exemple 4 avec le diéthylester comme solvant apolaire.

**Tableau 4 : Effets des fractions non volatile et volatile issues de l'oléorésine de Copaifera officinalis sur la métabolisation de la testostérone / la production de dihydrotestostérone (activité 5α-réductase)**

| Contrôle | Fraction non volatile | | | Fraction volatile | |
|---|---|---|---|---|---|
| | 0,3 µg/ml | 1 µg/ml | 3 µg/ml | 7,7 µg/ml | 23,1 µg/ml |
| 100 | 0 | -6% | -14% | +2% | +9% |
| | P=NS | P=NS | * P<0,05 | P=NS | P=NS |

| | | | | | |
|---|---|---|---|---|---|
| L'étude statistique est réalisée versus le groupe contrôle (test de Dunnett). | | | | | |

Il s'avère que l'activité de l'oléorésine de Copaifera officinalis est portée par la fraction non volatile, en effet aucune activité de la fraction volatile n'a été mise en évidence. L'inhibition de la 5α-réductase n'est que de 14% avec la fraction non volatile, mais cette réduction atteint la significativité statistique (p<0,05).

Une troisième série d'expériences a été mise en place afin de montrer que d'autres espèces de Copaifera, notamment C. multijuga présentaient également une activité intéressante sur l'inhibition de la 5α-réductase. Les inventeurs se sont focalisés sur la fraction non volatile, fraction portant l'activité inhibitrice. Les résultats sont résumés dans le tableau 5 ci-dessous.

**Tableau 5 : Effets des fractions non volatiles issues de l'oléorésine de Copaifera multijuga sur la métabolisation de la testostérone / la production de dihydrotestostérone (activité 5α-réductase ; n=2)**

| Contrôle | Fraction non volatile Copaifera multijuga | |
|---|---|---|
| | 1 µg/ml | 10 µg/ml |
| 100 | 0 | -31% |
| | P=NS | ** P<0,01 |

| | | |
|---|---|---|
| L'étude statistique est réalisée versus le groupe contrôle (test de Dunnett). La préparation des fractions volatile et non volatile est réalisée selon la méthode décrite dans l'exemple 4 avec le diéthylester comme solvant apolaire. | | |

Ces résultats montrent clairement que plusieurs espèces de Copaifera présentent un intérêt. En effet, la fraction non volatile de l'espèce C. multijuga atteint 31% d'inhibition de la 5α-réductase à 10µg/ml.

L'ensemble de ces résultats permet de conclure que l'oléorésine de Copaifera présente une activité inhibitrice sur la 5α-réductase extrêmement intéressante. Cette activité est portée par la fraction non volatile de l'oléorésine. Enfin les inventeurs ont également montré que cette activité est retrouvée sur plusieurs espèces de Copaifera.

### Exemple 2 : Préparation de l'oléorésine

L'écorce de tronc des arbres de *Copaifera officinalis* et/ou *Copaifera multijuga* et/ou *Copaifera reticulata* est entaillée afin de récupérer l'oléorésine. Celle-ci est ensuite homogénéisée puis stabilisée sous azote.

La matière active est composée à 100% d'oléorésine brute de tronc de Copaifera *officinalis* et/ou *Copaifera multijuga* et/ou *Copaifera reticulata.*

### Analyse LCMS d'une oléorésine de Copaifera officinalis

Chaque échantillon a été analysé par UHPLC-QTOFMS selon un gradient linéaire classique.

Séparation sur système Waters Acquity UHPLC.
- Colonne 100 x 2,1 mm, 1,7 µm, Acquity BEH C18 équipé d'une pré-colonne
- Phase mobile :
Phase mobile A : Eau LCMS grade + 0,1 % acide formique
Phase mobile B : Acétonitrile LCMS grade + 0.,1% acide formique

- Gradient :

| Temps (min) | %A | %B |
|---|---|---|
| 0-0,5 | 50 | 50 |
| 0,5-4 | 50→ 40 | 50 → 60 |
| 4-12 | 40→ 1 | 60 →99 |
| 12-15 | 1 | 99 |
| 15-15,5 | 1 →50 | 99→50 |
| 15,5-19 | 50 | 50 |

### Acquisitions :

### UV 220 nm

| Structure | m/z | Teneur dans l'oléorésine (% massique par rapport au poids de l'oléorésine) |
|---|---|---|
| Acide Copalique | 304 | 5,69 |
| Acide Hardwickiique | 316 | 0,61 |
| Acide Copaiferolique | 320 | 2,90 |

| | | |
|---|---|---|
| Acide Agathique et ester méthylique de l'acide 3 beta-hydroxyanticopalique | 334 | 2,35 |
| Ester diméthylique de l'acide agathendioique | 362 | 2,92 |
| Acide 7α-acetoxyhardwickiique | 374 | 0,79 |

### Exemple 3 : Préparation de la fraction non volatile

L'oléorésine obtenue selon l'exemple 2 est mise en suspension dans 10 volumes d'eau chauffée à 100 °C pendant 4 h pour réaliser une hydrodistillation. L'huile essentielle, volatile, est récupérée par condensation. Après l'hydrodistillation, le résidu de distillation est récupéré. Après séchage par lyophilisation ou autre moyen de séchage, le résidu constitue la fraction non volatile.

### Exemple 4 : Autre préparation possible de la fraction non volatile

Un volume d'oléorésine de Copaifera obtenue selon l'exemple 2 est diluée dans 8 à 10 volumes d'un solvant lipophile non miscible à l'eau (tel que le diéthyl éther, l'acétate d'éthyle). Cette solution est extraite par extraction liquide/liquide avec une solution de soude (NaOH) à 5 %. L'opération est répétée 3 fois avec 4 à 5 volumes de NaOH 5 %. La phase inférieure (phase aqueuse basique) est acidifiée par addition d'acide chlorhydrique (HCl) 1 N puis extraite par extraction liquide/liquide avec un solvant apolaire non miscible avec l'eau (comme du diéthyléther, l'acétate d'éthyle). La phase acétate d'éthyle est lavée à l'eau puis déshydratée sur Na2SO4. Après élimination du solvant par rotavapor ou autres moyens de séchage, le résidu sec obtenu correspond au mélange d'acides diterpéniques et/ou esters d'acides diterpéniques selon l'invention.

| Structure | m/z | Teneur massique dans la fraction non volatile |
|---|---|---|
| Acide Copalique | 304 | 28,45 |
| Acide Hardwickiique | 316 | 3,05 |
| Acide Copaiferolique | 320 | 14,52 |
| Acide Agathique Et ester méthylique de l'acide 3 beta-hydroxyanticopalique | 334 | 11,77 |
| Ester diméthylique de l'acide agathendioique | 362 | 14,58 |
| Acide 7α-acetoxyhardwickiique | 374 | 3,96 |

## Revendications

1. Oléorésine de Copaifera pour son utilisation en tant que médicament destiné au traitement et/ou à la prévention de l'hyperplasie bénigne de la prostate.

2. Oléorésine de Copaifera pour son utilisation selon la revendication 1, **caractérisée en ce que** l'oléorésine provient de l'espèce de Copaifera choisie parmi le groupe constitué de C. officinalis, C. multijuga, C. reticulata.

3. Fraction d'acides diterpéniques et/ou esters d'acides diterpéniques d'une oléorésine de Copaifera, **caractérisée en ce qu'**elle contient au moins 90% d'acides diterpéniques et/ou esters d'acides diterpéniques en poids pour son utilisation en tant que médicament destiné au traitement et/ou à la prévention de l'hyperplasie bénigne de la prostate.

4. Composition pharmaceutique **caractérisée en ce qu'**elle contient :
- comme agent actif une oléorésine de Copaifera ou une fraction d'acides diterpéniques et/ou d'esters d'acides diterpéniques d'une oléorésine de Cofaifera, ladite fraction comprenant au moins 90% d'acides diterpéniques et/ou esters d'acides diterpéniques en poids, et
- au moins un excipient pharmaceutiquement acceptable,
pour son utilisation comme médicament destiné au traitement et/ou la prévention de l'hyperplasie bénigne de la prostate.

5. Composition pharmaceutique pour son utilisation selon la revendication 4, **caractérisée en ce que** l'oléorésine provient de l'espèce de Copaifera choisie parmi le groupe constitué de C. officinalis, C. multijuga, C. reticulata.

6. Composition pharmaceutique pour son utilisation selon l'une des revendications 4 ou 5, **caractérisée en ce qu'**elle contient comme agent actif la fraction d'acides diterpéniques et/ou d'esters d'acides diterpéniques d'une oléorésine de Cofaifera.

7. Composition pharmaceutique pour son utilisation selon l'une des revendications 4 à 6, **caractérisée en ce qu'**elle se présente sous une forme adaptée à une administration par voie orale ou intraveineuse.

## Patentansprüche

1. Copaifera-Oleoresin zur Verwendung als Arzneimittel zur Behandlung und/oder Vorbeugung von gutartiger Hyperplasie der Prostata.

2. Copaifera-Oleoresin zur Verwendung nach Anspruch 1, **dadurch gekennzeichnet, dass** das Oleoresin von der Copaifera-Art stammt, die ausgewählt ist aus der Gruppe bestehend aus C. officinalis, C. multijuga und C. reticulata.

3. Fraktion von Diterpen-Säuren und/oder Diterpen-Säureestern eines Copaifera-Oleoresins, **dadurch gekennzeichnet, dass** sie mindestens 90 Gew.-% Diterpen-Säuren und/oder Diterpen-Säureester zur Verwendung als Arzneimittel zur Behandlung und/oder Vorbeugung von gutartiger Hyperplasie der Prostata enthält.

4. Pharmazeutische Zusammensetzung, **dadurch gekennzeichnet, dass** sie enthält:
- als Wirkstoff ein Copaifera-Oleoresin oder eine Fraktion von Diterpen-Säuren und/oder Diterpen-Säureestern eines Copaifera-Oleoresins, wobei diese Fraktion mindestens 90 Gew.-% Diterpen-Säuren und/oder Diterpen-Säureester enthält, und
- mindestens einen pharmazeutisch annehmbaren Hilfsstoff,
zur Verwendung als Arzneimittel zur Behandlung und/oder Vorbeugung von gutartiger Hyperplasie der Prostata.

5. Pharmazeutische Zusammensetzung zur Verwendung nach Anspruch 4, **dadurch gekennzeichnet, dass** das Oleoresin von der Copaifera-Art stammt, die ausgewählt ist aus der Gruppe bestehend aus C. officinalis, C. multijuga und C. reticulata.

6. Pharmazeutische Zusammensetzung zur Verwendung nach einem der Ansprüche 4 oder 5, **dadurch gekennzeichnet, dass** sie als Wirkstoff die Fraktion von Diterpen-Säuren und/oder Diterpen-Säureestern eines Copaifera-Oleoresins enthält.

7. Pharmazeutische Zusammensetzung zur Verwendung nach einem der Ansprüche 4 bis 6, **dadurch gekennzeichnet, dass** sie in einer Form vorliegt, die für die orale oder intravenöse Verabreichung geeignet ist.

## Claims

1. Copaifera oleoresin for use as a medicine for the treatment and/or prevention of benign prostatic hyperplasia.

2. Copaifera oleoresin for use according to claim 1, **characterised in that** the oleoresin comes from the Copaifera species selected from the group consisting of C. officinalis, C. multijuga, C. reticulata.

3. Fraction of diterpenic acids and/or diterpenic acid esters of a Copaifera oleoresin, **characterised in that** it contains at least 90 wt.% diterpenic acids and/or diterpenic acid esters for use as a medicine for the treatment and/or prevention of benign prostatic hyperplasia.

4. Pharmaceutical composition **characterised in that** it contains:
- as active agent, a Copaifera oleoresin or a fraction of diterpenic acids and/or diterpenic acid esters of a Copaifera oleoresin, said fraction comprising at least 90 wt.% diterpenic acids and/or diterpenic acid esters, and
- at least one pharmaceutically acceptable excipient,
for use as a medicine for the treatment and/or prevention of benign prostatic hyperplasia.

5. Pharmaceutical composition for use according to claim 4, **characterised in that** the oleoresin comes from the Copaifera species selected from the group consisting of C. officinalis, C. multijuga, C. reticulata.

6. Pharmaceutical composition for use according to one of claims 4 or 5, **characterised in that** it contains as active agent the fraction of diterpenic acids and/or diterpenic acid esters of a Copaifera oleoresin.

7. Pharmaceutical composition for use according to one of claims 4 to 6, **characterised in that** it is in a form suitable for oral or intravenous administration.
